(19) Europäisches Patentamt
European Patent Office
Office européen des brevets

(11) Publication number: **0 069 424**
**B1**

(12) # EUROPEAN PATENT SPECIFICATION

(45) Date of publication of patent specification: **29.05.85**

(51) Int. Cl.[4]: **C 07 C 45/64**, C 07 C 49/753, C 07 C 49/747

(21) Application number: **82200806.6**

(22) Date of filing: **30.06.82**

(54) **Process for the selective reduction of a 14, 17-dioxo-8,4-seco-steroid.**

(30) Priority: **07.07.81 NL 8103233**

(43) Date of publication of application:
**12.01.83 Bulletin 83/02**

(45) Publication of the grant of the patent:
**29.05.85 Bulletin 85/22**

(84) Designated Contracting States:
**CH DE FR GB IT LI NL SE**

(56) References cited:
**FR-A-1 511 392**
**GB-A-1 180 584**

**JOURNAL OF ORGANIC CHEMISTRY, vol. 45, 1980, pp. 582-588; N.COHEN et al.: "Asymetric reductions of alpha-beta-acetylenic ketones and acetophenone using lithium aluminum hydride complexed with optically active 1,3-amino alcohols"**

(73) Proprietor: **AKZO N.V.**
**Velperweg 76**
**NL-6824 BM Arnhem (NL)**

(72) Inventor: **Peters, Jacobus Albertus Maria**
**Spiegelstraat 14**
**Oss (NL)**
Inventor: **Zeelen, Filippus Johannes**
**Floraliastraat 2**
**Heesch (NL)**
Inventor: **Van Vliet, Nicolaas Pieter**
**Ribeslaan 16**
**Rhenen (NL)**

(74) Representative: **van 't Holt, Hendrik et al**
**Postbus 20**
**NL-5340 BH Oss (NL)**

(56) References cited:
**CHEMISCHE BERICHTE, vol. 108, 1975, pp. 2665-2672; G.HAFFER et al.: "Totalsynthese optisch asktiver Steroide, X. Reduktion von 3-Methoxy-8,14-seco-1,3,5(10)9-östratetraen-14,17-dion mit chiralen komplexen Hydriden"**

**EP 0 069 424 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to a process for the selective reduction of a 14,17-dioxo-8,14-seco-1,3,5(10),9(11)-oestratetraene to the corresponding 14,17-ketol with the aid of a chiral reducing agent.

The reduction of such a diketone to the corresponding ketol forms an important intermediate step in the total synthesis of 19-nor-steroids by Torgov's method.

In the original Torgov synthesis of 19-norsteroids (Tetrahedron Letters (1963), 1553) 3-methoxy-8,14-seco-1,3,5(10),9(11)-oestratetraene-14,17-dione is condensed under the influence of an acid to 3-methoxy-1,3,5(10),8,14-oestrapentaen-17-one. During this step a chiral centre forms which gives rise to the formation of a racemate. By resolution it is possible to isolate from the racemate the steroid with natural configuration. The unnatural steroid (the enantiomer), for which as yet no practical application has been found, has to be discarded.

Many attempts have been made to arrive at a stereoselective modification of the Torgov-synthesis. Detailed investigations have been made into selective reduction of the 14,17-dione to the corresponding ketol followed by ring closure of the 8,14-seco-compound.

Details are given in J. Org. Chem. *33*, 3126 (1968) of the reduction of 3-methoxy-8,14-seco-1,3,5(10),9(11)-oestratetraene-14,17-dione by means of $Li(O\text{-}t\text{-}butyl)_3AlH$ to the corresponding ketol.

The racemic trans-ketol turns out to be the main product (85—90%), and the racemic cis-ketol is the by-product. Thus the reduction exhibits a high diastereoselectivity.

In this specification "ketol" signifies the oxo-alcohol obtained by partial reduction of the 14,17-dione. "Cis" and "trans" denote the position of the hydroxyl group with respect to the alkyl group at position 13 in the 8,14-seco-steroid.

From the racemic trans-ketol obtained by partial reduction as described above it is possible to obtain the trans-ketol with natural configuration by resolution via the hemisuccinate and the quinine salt thereof. The undesirable isomers can be converted back into the initial dione by oxidation. Cyclisation of the acetylated trans-ketol with natural configuration gives the natural 3-methoxy-1,3,5(10)8,14-oestrapentaen-17α-ol 17-acetate.

A similar route, but using $NaBH_4$ as reducing agent is described in Chem. Pharm. Bulletin, *21*, 107 (1973), in which at the same time it is pointed out that reduction of the 13-ethyl-analogue (the 18-methyl-homologue) also supplies mainly the trans-ketol.

A further development in the stereoselective reduction of 3-methoxy-8,14-seco-1,3,5(10),9(11)-oestratetraene-14,17-dione is described in Chem. Ber. *108*, 2665 (1975), viz. the reduction with chiral reducing agents. Lithium aluminiumhydride complexes with chiral alcohols, optionally combined with achiral alcohols, were employed as chiral reducing agents. The following chiral alcohols were used: (−)-butane-2,3-diol, (+)-butane-2,3-diol, (−)-1,4-bis(dimethylamino)-(2S,3S)-butane-2,3-diol, (+)-pseudo-ephedrine, (−)-ephedrine.

Benzyl alcohol, isopropanol, t.-butyl alcohol and the methyl ether of glycol were used as achiral alcohols. Although, with the different complexes, a reasonable diastereoselectivity (trans/cis-ratio 7:3) was obtained, the enantioselectivity was only 23% e.e. (enantiomeric excess) or less for the trans-ketol and only 7% e.e. or less for the cis-ketol. The best results were obtained with a complex of $LiAlH_4$, (−)-ephedrine and benzylalcohol. An optically-active mixture of the trans-ketol and the cis-ketol was obtained with a ratio 7:3 using this complex, whilst for the trans-ketol the enantiomeric ratio was 61.5/38.5. In this case apart from the relatively simple cis/trans separation, a difficult separation of enantiomers is furthermore always required in order to obtain optically pure compounds.

It should also be pointed out that for cyclisation a cis-ketol is more suitable than a trans-ketol. A cis-ketol can be cyclised directly with acid to the corresponding 1,3,5(10),8,14-gonapentaene. In the presence of acid a trans-ketol will initially give rise to the formation of a 9,14-spiro-ether which subsequently can be cyclised under somewhat more rigorous conditions to the desired 1,3,5(10),8,14-gonapentaene. In order to prevent the spiro-ether formation a trans-ketol is best initially acetylated, after which the acetylated trans-ketol is cyclised with acid to the corresponding 17-acetoxy-1,3,5(10),8,14-gonapentaene. Hydrolysis of the 17-acetoxy group supplies the free alcohol.

It has been surprisingly established that using a chiral reducing agent consisting of a complex of an alkali metal aluminium hydride with a special chiral alcohol, optionally combined with an achiral alcohol or phenol, 8,14-seco-1,3,5(10),9(11)-oestratetraene-14,17-diones can be reduced to the corresponding 14,17-ketols with an enantioselectivity (enantiomeric ratio) which is so much more favourable than that obtainable hitherto that it is possible to isolate optically pure or almost optically pure ketols from the reaction mixture without having to resolve racemates or other enantiomeric mixtures, whilst in addition a high, i.e. favourable diastereoselectivity (trans/cis- or cis/trans-ratio) is obtained.

To clarify the concepts "diastereoselectivity" and "enantioselectivity" reference is made to Angew. Chemie (Int. Ed.) *10* (12), 871 (1971).

Thus the invention relates to a process for the selective reduction of an 8,14-seco-1,3,5(10),9(11)-oestratetraene-14,17-dione to the corresponding 14,17-ketol with the aid of a chiral reducing agent and is characterised in that such a 14,17-dione is reduced by means of a complex alkali metal aluminium-hydride having the formula:

$$M\,Al(OR_1)_m(OR_2)_n\,H_{4-m-n},$$

in which

M represents an alkali metal;

$OR_1$ is derived from the chiral alcohol (+)-(2S,3R)-4-dimethylamino-3-methyl-1,2-diphenyl-2-butanol or the enantiomer thereof with the (−)-(2R,3S)-configuration;

$OR_2$ is derived from an achiral organic hydroxy compound having 1—10 C-atoms;

m = 1—3; n = 0—2; and m + n ≤ 3.

M can for example be lithium, sodium or potassium and is preferably lithium.

The chiral reducing agent based on the chiral alcohol in the (+)-(2S,3R) form is as such known from J. Org. Chem. *38*, 1870 (1973). In the asymmetric reduction described therein of achiral ketones to chiral alcohols, admittedly a certain level of enantioselectivity is achieved, but optically pure or almost optically pure alcohols are not obtained.

The achiral organic hydroxy compound optionally employed in the complex can be alcohol such as methanol, ethanol, propanol, isopropanol, butanol, isobutanol, tert.-butanol, benzylalcohol, glycol monomethylether and the like, or a phenol substituted or unsubstituted by alkyl groups such as phenol, 3,5-0dimethyl phenol, and the like. Since the use of groups $OR_2$ in the complex offers no advantages, as compared with complexes without $OR_2$ groups, and sometimes even gives poorer results as regards asymmetric induction and/or yield, n is preferably 0.

In the formula $M\ Al(OR_1)_m(OR_2)_n\ H_{4-m-n}$, m and n relate to the number of mol equivalents of the groups $OR_1$ and $OR_2$ per mol complex, i.e. m and n do not need to definitely be whole numbers, but can have each value within the specified limits.

Preferably m is 2—3, but especially 2—2.5.

The complex is obtained by allowing lithium aluminium hydride to react in a solvent, normally diethylether, at a temperature of 0—30°C whilst being stirred with the chiral alcohol and optionally the achiral hydroxy compound in the desired molar ratio.

The process in accordance with the invention is applied preferably to 14,17-dioxo-8,14-seco steroids having the formula:

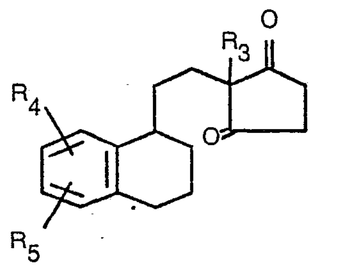

in which

$R_3 = CH_3$ or $C_2H_5$

$R_4$ = H, alkyl, OH or alkoxy; and

$R_5$ = H, alkyl, OH or alkoxy.

Concerning the substituents $R_4$ and $R_5$ the following can be stated:

Preferably one of the substituents $R_4$ and $R_5$ is OH or alkoxy, such as methoxy, and is then located at the 3-position. The other substituent is then for example H, methyl or methoxy and is then located at the 1-position, the 2-position or the 4-positon. Preferably $R_4$ is H and $R_5$ is 3-methoxy, because such initial products via the reduction in accordance with the invention, the cyclisation of the ketol thus obtained and a number of reduction steps lead to oestradiol ($R_3 = CH_3$) and important 19-nor-steroids.

Reduction of the diketones mentioned above leads in principle to a mixture of 4 stereoisomeric ketols (2 racemates) with partial structure:

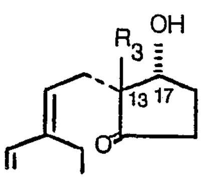

(13R, 17R) ;

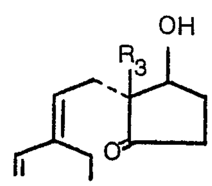

(13R, 17S) ;

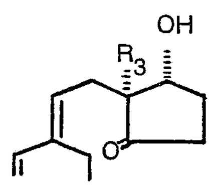

(13S, 17R) resp.

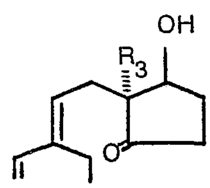

(13S, 17S)

These 4 ketols possess the configuration specified underneath the respective partial structures. The application of the chiral reducing agent in accordance with the invention leads surprisingly exclusively, or almost exclusively, to a mixture of two stereoisomeric ketols which are not mirror images of each other, but are a trans-ketol and a cis-ketol. Dependent on the application in the chiral reducing agent of the (+)-(2S,3R)-amino-alcohol, or the (−)-(2R,3S)-amino-alcohol, the reduction gives either a (optically active)

mixture of the trans- and the cis-ketol both with the 17R-configuration or a (optically active) mixture of the trans- and the cis-ketol both with the 17S-configuration.

When $R_3 = CH_3$, then the use of the (+)-(2S,3R)-amino-alcohol in the chiral reducing agent gives mainly the trans-ketol with (13R,17R)-configuration with as by-product also the cis-ketol with (13S,17R)-configuration. In this case the trans/cis ratio is 7 to 8:3 to 2.

The mixture thus obtained of trans- and cis-ketol can be separated in the usual way, e.g. by fractional crystallisation or by means of chromatography.

The 13-methyl-(17R)-trans-ketol can subsequently be cyclised in the known manner, preferably after acetylation, to give the corresponding 13-methyl-1,3,5(10),8,14-gonapentaene with natural configuration. The 13-methyl-(17R)-cis-ketol can, possibly after acetylation, be cyclised in a similar manner to give the enantiosteroid or be reconverted by oxidation into the 14,17-dione, which can then be employed once more for reduction. Having regard to the hitherto low demand for ent-steroids, preference will be given to conversion into the 14,17-dione.

When using the enantiomeric (−)-amino-alcohol in the chiral reducing agent, the enantiomeric ketols are obtained in the same trans/cis ratio, i.e. the (13S,17S)-trans-ketol and the (13R,17S)-cis-ketol.

When $R_3 = C_2H_5$, then remarkably during reduction in accordance with the invention the diastereoselectivity appears to be completely reversed whilst maintaining the 100% or almost 100% enantioselectivity. When using the (+)-(2S,3R)-amino-alcohol in the chiral reduction agent, in this case it is precisely essentially the cis-ketol with (13S,17R)-configuration which is obtained with also as by-product, the transketol with (13R,17R)-configuration. In this case the cis/trans ratio is 7 to 8:3 to 2.

Cyclization of the main product (the 13-ethyl-(17R)-cis-ketol) leads to the corresponding 13-ethyl-1,3,5(10),8,14-gonapentaene with unnatural configuration. The byproduct gives a steroid with natural configuration. By now employing the (−)-(2R,3S)-amino-alcohol in the chiral reducing agent, for $R_3 = C_2H_5$, during the reduction of the 14,17-dione mainly the cis-ketol with (13R,17S)-configuration is obtained and the trans-ketol with (13S,17S)-configuration, and now once more with a cis-trans ratio of 7 to 8:3 to 2.

Cyclization of the 13-ethyl-(17S)-cis-ketol supplies the corresponding 13-ethyl-1,3,5,(10), 8,14-gonapentaene with natural configuration. The by-product (the trans-ketol) can, preferably before cyclization of the cis-ketol, be isolated in the normal way and, by oxidation, be converted back into the diketone, which can be employed once again in the reduction step. Naturally the (17S)-trans-ketol can also be cyclized after acetylation to give a 13-ethyl-gonapentaene with unnatural configuration if there is a need for this.

A surprising advantage of the 13-ethyl series is that the 13-ethyl-1,3,5(10),8,14-gonapentaene-17β-ol or its 17-acetate is obtained as main product from the 13-ethyl-(17S)-cis-ketol. As has already been pointed out, a cis-ketol is more suitable for cyclization than is a trans-ketol. A further advantage is also that the natural gonapentaene obtained with the 13-ethyl-group and the 17-hydroxy group in the cis position (13β-ethyl-17β-OH) during the following reduction of the $\Delta^{14}$ double bond (catalytic reduction with Pd on $CaCO_3$) gives a higher yield of desirable 14α-compound than would be the case with the trans-compound (13β-ethyl-17α-OH).

The process in accordance with the invention is characterised by its versatility. Both in the 13-methyl and the 13-ethyl series it is possible, at will, to obtain the natural or the unnatural steroid as main product by employing the chiral reducing agent based on the (+)-(2S,3R)-amino-alcohol or the (−)-(2R,3S)-amino-alcohol or conversely. The main product can, without a great deal of difficulty, be separated from the by-product. Resolution of a racemate or other mixture of enantiomers is not necessary. By-products are not lost, but can be converted once more into initial starting products. The chiral alcohol used in the chiral reducing agent is similarly not lost, but can be recovered from the mother liquor by the addition of acid without racemization and can hence be employed once more.

From the above it is clear that the invention can also be characterised as a method for manufacturing optically pure 14,17-ketols from 8,14-seco-1,3,5(10),9(11)-oestratetraenes without the need for racemate or enantiomeric mixture resolution, by reducing the corresponding 14,17-dione with the above-mentioned chiral reducing agent.

The following statements can also be made with regard to the reaction conditions of the reduction stage:

As medium for the reduction a suitable solvent is selected, usually ether (diethyl-ether), but the reaction can also be undertaken in tetrahydrofuran, toluene or in alkanes such as pentane or hexane.

Reduction is undertaken at low temperatures, normally between −50°C and −100°C and preferably at a temperture between −60°C and −80°C.

The amount of reducing agent with respect to the amount of dione is at least equimolar and is normally, for practical reasons, not more than 5 mol per mol dione. Preferably a quantity of 2 mol reducing agent per mol dione is employed.

Of the initial 14,17-diones, the 3-methoxy-8,14-seco-1,3,5(10),9(11)-oestratetraene-14,17-dione and the 13-ethyl-analogue are described in the literature. 14,17-Diones with a substitution in ring A differing therefrom can be produced in accordance with a known method using the Torgov synthesis by reaction of a 2-$R_3$-cyclopentane-1,3-dione ($R_3 = CH_3$ or $C_2H_5$) with a 1-vinyl-1-tetralol with the desired $R_4$,$R_5$ substitution in the "pro-A"-ring, e.g. 6-methoxy-8-methoxy-1-vinyl-1,2,3,4-tetrahydro-1-naphthol or 5,6-dimethoxy-1-vinyl-1,2,3,4-tetrahydro-1-naphthol.

The optically pure 1,3,5(10),8,14-gonapentaenes obtained by cyclization of the optically pure ketols can be converted in the conventional manner into important known steroids.

First of all the $\Delta^{14}$ double bond is reduced, catalytically, with Pd on $CaCO_3$ as catalyst, whereby the desired 14α-steroid is obtained almost exclusively. The subsequent reduction of the $\Delta^8$ double bond with lithium in $NH_3$ leads to oestradiol or derivatives thereof.

A Birch reduction of the aromatic A-ring then gives access to 3-keto-$\Delta^4$-oestrenes or 3-keto-$\Delta^{5(10)}$-oestrenes. The introduction of an ethynyl group at position 17 then gives known progestagens such as norethisteron and norethynodrel. In this way, in the 13-ethyl-series, we arrive at the known d-norgestrel.

The invention will now be illustrated with the aid of the following examples without these restricting its scope.

Example I

A solution of 34.0 g (120 mmol) of (+)-2S,3R)-4-dimethylamino-3-methyl-1,2-diphenyl-2-butanol in 90 ml dry ether was added dropwise to a stirred suspension of 1.96 g (52 mmol) $LiAlH_4$ in 870 ml of dry ether at 0°C under nitrogen. The mixture obtained was stirred for 5 minutes at 0°C and then cooled down to −75°C. A solution of 7.16 g (24 mmol) 3-methoxy-8,14-seco-1,3,5(10),9(11)-oestratetraene-14,17-dione in 150 ml of dry ether was added dropwise at such a rate that the temperature did not rise above −70°C. The mixture obtained was then stirred for 30 minutes at −75°C, after which about 40 ml of water was added carefully drop-wise. After heating up to 0°C it was acidified with cold 2 N HCl, water was added and the mixture was extracted with ether. The ether extract was washed until neutral with water and dried on anhydrous $MgSO_4$.

The combined water layers were rendered alkaline with 3 N KOH, by means of which the precipitated amino-alcohol could be recovered quantitatively and without racemization.

Concentration of the ether extract under reduced pressure gave a residue on drying of 7.05 g, in accordance with the $^1H$-NMR spectrum about 4:1 trans-: cis-ketol, each consisting of more than 95% of the enantiomer (determined by means of a chiral $^1H$-NMR-shift reagent).

By crystallisation from diisopropyl ether 4.45 g was obtained of the trans-ketol 17α-hydroxy-3-methoxy-8,14-seco-1,3,5(10),9(11)-oestratetraen-14-one with a melting point of 100—102° $[\alpha]_D^{20}$ −46° (c = 1, dioxane). The mother liquor was filtered in 1,000 ml ethylene chloride-acetone mixture (98:2) over a column of 100 g silicagel and then recrystallised out of methylene chloride-diisopropyl ether. 1.01 g of the cis-ketol 14β-hydroxy-3-methoxy-8,14-seco-1,3,5(10),9(11)-oestratetraen-17-one was obtained with a melting point of 110—112°C and $[\alpha]_D^{20}$ +37° (c = 1, dioxane).

The ketol obtained initially (4.45 g) was dissolved in 40 ml of dry pyridine and under nitrogen at room temperature 8 ml of acetic anhydride was added. The mixture was stirred for 16 hours at room temperature and was subsequently poured into a mixture of ice and water. After 3 hours stirring extraction with ether was carried out. The ether extract was washed consecutively with cold 2 N HCl, water and with $NaHCO_3$ solution. After drying on anhydrous $MgSO_4$ and evaporation of the solvent, this gave the 17α-acetoxy-3-methoxy-8,14-seco-1,3,5(10),9(1)-oestratetraen-14-one (5.00 g). Without further purification this compound was dissolved in 200 ml benzene, 320 mg p-toluene sulphonic acid was added and the mixture was boiled for 30 minutes using an azeotropic water separator. After cooling down the mixture was washed with $NaHCO_3$-solution and subsequently dried on anhydrous $MgSO_4$. The solvent was evaporated and the residue was recrystallised out of ethanol, which gave 4.01 g of 3-methoxy-1,3,5(10),8,14-oestrapentaen-17α-ol 17-acetate was melting point 127—128°C and $[\alpha]_D^{20}$ −183° (c = 1, $CHCl_3$).

Analogous with the ketol which had been isolated was also acetylated and then cyclised by treating it with p-toluene sulphonic acid. Recrystallization of the resultant product from ethanol gave 0.71 g ent-3-methoxy-1,3,5(10),8,14-oestrapentaen-17β-ol 17-acetate with melting point 87—89°C and $[\alpha]_D^{20}$ +180° (c = 1, $CHCl_3$).

Example II

The crude mixture of trans- and cis-ketol (7.05 g, about 4:1 trans/cis) obtained in accordance with the procedure in example I was acetylated and then cyclized with p-toluene sulphonic acid as described in example I for the separate ketols. The mixture thus obtained was separated via column chromatography over 200 g silicagel using hexane-ethyl acetate 98:2. Recrystallisation of the products obtained from ethanol gave 4.08 g 3-methoxy-1,3,5(10),8,14-oestrapentaene-17α-ol 17-acetate with melting point 127—128°C and $[\alpha]_D^{20}$ −183° (c = 1, $CHCl_3$) as main product. In addition 0.75 g of ent-3-methoxy-1,3,5(10),8,14-oestrapentaen-17β-ol 17-acetate was obtained with melting point of 87—89°C and $[\alpha]_D^{20}$ +180° (c−1, $CHCl_3$).

Example III

The procedures in example I and example II were repeated, the only difference being the (+-(2S,3R)-4-dimethylamino-3-methyl-1,2-diphenyl-2-butanol was replaced by an equal weight of its enantiomer (−)-(2R,3S)-4-dimethylamino-3-methyl-1,2-diphenyl-2-butanol. In this way the enantiomers of the products specified in examples I and II were obtained, these being: 14α-hydroxy-3-methoxy-8,14-seco-1,3,5(10),9(11)-oestratetraen-17-one (4.45 g) with melting point 100—102°C and $[\alpha]_D^{20}$ +46°C (c = 1, dioxane); 17β-hydroxy-3-methoxy-8,14-seco-1,3,5(10),9,(11)-oestratetraen-14-one (1.0 g) with melting

point of 110—112°C and $[\alpha]_D^{20}$ −37° (c = 1, dioxane); ent-3-methoxy-1,3,5(10),8,14-oestrapentaen-17α-ol 17-acetate (4.0 g) with melting point of 127—128°C and $[\alpha]_D^{20}$ + 183° (c = 1,$CHCl_3$); 3-methoxy-1,3,5(10),8,14-oestrapentaen-17β-ol 17-acetate (0.7 g) with melting point of 87—89°C and $[\alpha]_D^{20}$ −180° (c = 1, $CHCl_3$).

Example IV

a) 19.7 ml (29.7 g; 0.28 mol) of vinyl bromide dissolved in 70 ml of dry tetrahydrofuran was added dropwise to a mixture of 6.0 g (0.25 mol) magnesium, 7.5 ml dry tetrahydrofuran and a small crystal of iodine at such a rate that the mixture boiled gentley. Subsequently stirring was carried out for a further 30 minutes at room temperature, after which a solution of 11.8 g (62 mmol) 6-methoxy-8-methyl-1,2,3,4-tetrahydronaphthalen-1-one in 22.5 ml of dry tetrahydrofuran and 90 ml of dry ether was added dropwise, the temperature of the reaction mixture being maintained at 20°C. The mixture was stirred for 2 further hours at room temperature after which, subject to ice cooling, a solution of 16.6 g $NH_4Cl$ in 33 ml of water was added dropwise. The mixture obtained was diluted with water and extracted with ether. The ether extract was washed with saturated NaCl solution and dried on anhydrous $Na_2SO_4$. Evaporation of the solvent supplied the crude vinyl carbinol (15.8 g) which was employed without purification. It was placed in 50 ml dry ether and added dropwise to a suspension of 4.9 g (64 mmol) thioureum in 50 ml glacial acetic acid which was mintained at 20°C. The mixture was stirred for a further 16 hours at room temperature and then concentrated as far as possible under vacuum. The residue was boiled up for some minutes with 150 ml of dry ether, after which the crystalline deposit was drawn off and dried. The yield was 12.8 g 6-methoxy-8-methyl-1,2,3,4-tetrahydronaphthylidene-ethyl isothiouronium acetate with a melting point of 147—151°C.

b) A mixture of 10.0 g (30 mmol) of the isothiouronium salt obtained, 10.0 g (86 mmol) of 2-methyl-1,3-cyclopentanedione, 150 ml methanol and 150 ml water was boiled for 45 minutes under nitrogen. After cooling down to room temperature the greater part of the methanol was removed under reduced pressure. Then extraction was carried out with ethyl acetate. The extract was washed with saturated $NaHCO_3$ and with saturated NaCl solution and dried on anhydrous $MgSO_4$. Evaporation gave a yellow oil which was chromatographed over 200 g silicagel with hexane-ethyl-acetate-mixture 7:3 as elution agent. This gave 7.4 g of 3-methoxy-1-methyl-8,14-seco-1,3,5(10),9(11)-oestratetraen-14,17-dione as a colourless oil.

c) At 0°C under nitrogen a solution of 39.2 g (138 mmol) (−)-(2R,3S)-4-dimethylamino-3-methyl-1,2-diphenyl-2-butanol in 100 ml of dry ether was added dropwise to a stirred solution of 2.30 g (61 mmol) $LiAlH_4$ in 1000 ml of dry ether. The resultant mixture was stirred for a further 5 minutes at 0°C and then cooled down to −75°C. A solution of the 3-methoxy-1-methyl-8,14-seco-1,3,5(1),9(11)-oestratetraen-14,17-dione (7.40 g, 24 mmol) obtained in (b) in 170 ml dry ether was added dropwise at such a rate that the temperature did not rise above −70°C. After stirring had been continued for 30 minutes at −75°C, the mixture was worked up as described in example I. This gave a yellow oil (7.23 g) with $[\alpha]_D^{20}$ +33° (c = 1, $CHCl_3$) according to the $^1$H-NMR analysis roughly a 4:1 mixture of trans- and cis-ketol, each consisting of more than 90% of one enantiomer. This mixture was acetylated and subsequently cyclized with p-toluene sulphonic acid as described in example II. The resultant mixture was separated via column chromatography (200 g silicagel; elution agent hexane-ethyl acetate 98:2) and the products were recrystallized out of methanol. This gave 4.0 g ent-3-methoxy-1-methyl-1,3,5(10),8,14-oestrapentaen-17α-ol 17-acetate with a melting point 147—153°C and $[\alpha]_D^{20}$ −4.7° (c = 1, $CHCl_3$) plus 1.1 g 3-methoxy-1-methyl-1,3,5(10),8,14-oestrapentaen-17β-ol 17-acetate with melting point 94—95°C and $[\alpha]_D^{20}$ +2.7°n (c = 1, $CHCl_3$).

Example V

In the same manner as described in example IV, 5,6-dimethoxy-1,2,3,4-tetrahydronaphthalen-1-one was converted via the 5,6-dimethoxy-1,2,3,4-tetrahydronaphthylidene-ethyl isothiouronium-acetate into 3,4-dimethoxy-8,14-seco-1,3(10),9(11)-oestratetraene-14,17-dione.

Then this dione (8.20 g, 25 mmol) was reduced as described in example I with the reagent formed from 2.10 g (55 mmol) $LiAlH_4$ and 35.75 g (125 mmol) (−)-(2R,3S)-4-dimethylamino-3-methyl-1,2-diphenyl-2-butanol. After treatment as described in example I the reaction product was recrystallised out of methylene chloride-diisopropylether, this giving 4.83 g 14α-hydroxy-3,4-dimethoxy-8,14-seco-1,3,5(10),9(11)-oestratetraene-17-one with a melting point of 117—119°C $[\alpha]_D^{20}$ +69° (c = 0.87; $CHCl_3$) and >98% of one enantiomer in accordance with $^1$H-NMR analysis.

Chromatography of the mother liquor over silicagel with methylene chloride-acetone 98:2 as elution agent, followed by crystallisation out of methylene chloridediisopropyl ether gave 1.30 g of 17β-hydroxy-3,4-dimethoxy-8,14-seco-1,3,5(10),9(11)-oestratetraen-14-one with melting point 88—91°C, $[\alpha]_D^{20}$ −56° (c = 1.18, $CHCl_3$) and >98% of one enantiomer in accordance with $^1$H-NMR analysis. Both ketols were acetylated and subsequently cyclized with p-toluene sulphonic acid as described in example I. Recrystallisation of the two reaction products gave respectively: 4.74 g ent-3,4-dimethoxy-1,3,5(10),8,14-oestrapentaen-17α-ol 17-acetate with melting point 188—189°C, $[\alpha]_D^{20}$ +162° (c = 0.93, $CHCl_3$), >98% an enantiomer ($^1$H-NMR-analysis) and 1.5 g, 3,4-dimethoxy-1,3,5(10),8,14-oestrapentaene-17β-ol 17-acetate with $[\alpha]_D^{20}$ −157° (C = 0.9, $CHCl_3$), >98% of one enantiomer ($^1$H-NMR-analysis).

Example VI

a) 7.50 g (24 mmol) 13-ethyl-3-methoxy-8,14-seco-1,3,5(10),9(11)-gonatetraene-14,17-dione was reduced as described in example I with the reagent formed from 1.96 g (52 mmol) $LiAlH_4$ and 34.0 g (120 mmol) (−)-(2R,3S)-4-dimethylamino-3-methyl-1,2-diphenyl-2-butanol and then treated as indicated in example I. The product obtained was, according to the $^1$H-NMR analysis, a mixture of cis- and trans-ketol in a ratio of roughly 4:1, whereby each ketol consisted of more then 95% of one enantiomer. Recrystallisation of this product from diisopropyl ether gave 4.50 g of the cis.ketol 13-ethyl-17β-hydroxy-3-methoxy-8,14-seco-1,3,5(10),9(11)-gonatetraen-14-one with melting point 86—88°C and $[\alpha]_D^{20}$ +13° (c = 1, dioxane).

b) The ketol obtained in (a) was acetylated and then cyclized with p-toluene sulphonic acid as described in example I. The product was recrystallised out of methanol, which gave 4.2 g 13-ethyl-3-methoxy-1,3,5(10),8,14-gonapentaen-17β-ol 17-acetate with melting poin 77—78°C and $[\alpha]_D^{20}$ −199° (c = 1, $CHCl_3$).

c) The mother liquor which remained after crystallisation of the cis-ketol in (a), (3.0 g) was mixed in 15 ml dry dimethylsulphoxide, after which 15 ml of benzene, 0.8 ml pyridine and 0.4 ml trifluoroacetic acid were added.

Subsequently 6.2 g dicyclohexylcarbodiimide was added and the mixture was stirred for 16 hours at room temperature. 250 ml ether was added, followed by a solution of 2.70 g oxalic acid in 25 ml methanol. After gas evolution had ceased 250 ml water was added and the mixture was filtered. The organic phase was washed with $NaHCO_3$-solution and with water, dried on anhydrous $Na_2SO_4$, filtered over 30 g silicagel and evaporated to dryness under reduced pressure. The residue on drying (2.90 g) consisted of practically pure 13-ethyl-3-methoxy-8,14-seco-1,3,5(10),9(11)-gonatetraene-14,17-dione, suitable for recirculation to the reduction process.

Example VII

At 0°C under nitrogen 2.9 ml (50 mmol) of absolute ethanol was added dropwise slowly to a stirred suspension of 1.90 g (50 mmol) $LiAlH_4$ in 850 ml of dry ether, after 15 minutes subsequent stirring followed by a solution of 28.3 g (100 mmol) (+)-(2S,3R)-4-dimethylamino-3-methyl-1,2-diphenyl-2-butanol in 70 ml of dry ether. After stirring for 5 minutes at 0°C this reagent was then used to reduce, at −70°C, 7.16 g (24 mmol) of 3-methoxy-8,14-seco-1,3,5(10),9(11)-oestratetraene-14,17-dione as described in example I. The mixture, treated in an analogous manner, consisting according to the $^1$H-NMR analysis of a mixture of trans- and cis-ketol in a ratio of about 7:3; whereby each ketol consisted of more than 95% of one enantiomer.

4.25 g 17α-hydroxy-3-methoxy-8,14-seco-1,3,5(10),9(11)-oestratetraen-14-one with a melting point of 100-102°C and $[\alpha]_D^{20}$ −46° (c = 1, dioxane) plus 1.10 g 14β-hydroxy-3-methoxy-8,14-seco-1,3,5(10),9(11)-oestratetraen-17-one with melting point 110—112°C and $[\alpha]_D^{20}$ +37° (c = 1, dioxane) were isolated from this in the manner described in example I.

Example VIII

A solution of 34.0 g (120 mmol (+)-(2S,3R)-4-dimethylamino-3-methyl-1,2-diphenyl-2-butanol in 200 ml dry toluene was added dropwise at 20°C under nitrogen to a stirred suspension of 2.27 g (60 mmol) $LiAlH_4$ in 600 ml of dry toluene. The mixture was stirred for 15 minutes at 20°C and then cooled down to −60°C. At this temperature a solution of 5.96 g (20 mmol) 3-methoxy-8,14-seco-1,3,5(10),9(11)-oestratetraene-14,17-dione in 150 ml of dry toluene was added dropwise. The mixture obtained was stirred for a further 30 minutes at −50°C after which about 40 ml of water was carefully added dropwise. After treatment as specified in example I similar results were obtained.

Example IX

In the same manner as described in example IV 6,7-dimethoxy-1,2,3,4-tetrahydronaphthalen-1-one was converted via 6,7-dimethoxy-1,2,3,4-tetrahydronaphthylidene-ethyl-isothiouronium acetate into 2,3-dimethoxy-8,14-seco-1,3,5(10),9(11)-oestratetraene-14,17-dione, this dione was reduced with the chiral reducing agent based on the (+)-amino-alcohol to the trans-ketol 17α-hydroxy-2,3-dimethoxy-8,14-seco-1,3,5(10),9(11)-oestratetraen-14-one and the cis-ketol 14β-hydroxy-2,3-dimethoxy-8,14-seco-1,3,5(10),9(11)-oestratetraen-17-one (trans:cis = 4:1) and the ketols were cyclized to give respectively 2,3-dimethoxy-1,3,5(10),8,14-oestrapentane-17α-ol 17-acetate and ent-2,3-dimethoxy-1,3,5(10),8,14-oestrapentaene 17β-ol 17-acetate.

**Claims**

1. Process for the selective reduction of an 8,14-seco-1,3,5(10),9(11)-oestratetraene-14,17-dione to the corresponding 14,17-ketol by means of a chiral reducing agent, characterised that such a 14,17-dione is reduced with the aid of a complex alkali metal aluminium hydride having the formula: $MA1(OR_1)_m(OR_2)_n H_{4-m-n}$, in which M represents an alkali metal: $OR_1$ is derived from the chiral alcohol (+)-(2S,3R)-4-dimethylamino-3-methyl-1,2-diphenyl-2-butanol or the enantiomer thereof with the (−)-(2R,3S)-configuration; $OR_2$ is derived from an achiral organic hydroxy compound having 1—10 C-atoms; m = 1—3; n = 0—2; and m + n≤3.

2. Process as in claim 1 characterised by the fact that M represents lithium.

3. Process as in claims 1 or 2 characterised in that a 14,17-dioxo-8,14-seco-steroid having the formula:

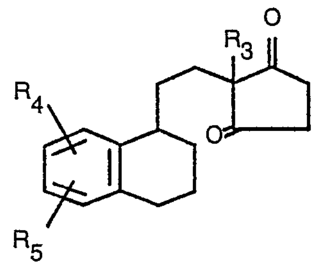

in which $R_3 = CH_3$ or $C_2H_5$; $R_4$ = H, alkyl, OH or alkoxy and $R_5$ = H, alkyl, OH or alkoxy, is reduced.

4. Process as in claim 3 characterised in that for $R_3 = CH_3$ the diketone is reduced with the chiral reducing agent based on the chiral alcohol having the (+)-(2S,3R) configuration.

5. Process as in claim 3 characterised in that for $R_3 = C_2H_5$ the diketone is reduced with the chiral reducing agent based on the chiral alcohol having the (−)-(2R,3S) configuration.

6. Process as in claims 3—5 characterised in that $R_4$ = H and $R_5$ = 3-methoxy.

7. Process as in claims 1—6 characterised by n = 0.

8. Process as in claims 1—7, characterised in that m = 2—3, preferably 2—2.5.

9. Process as in claims 1—8, characterised in that the reduction is carried out at a temperature between −50°C and −100°C, preferably between −60°C and −80°C.

10. Process as in claims 1—9, characterized in that per mol 14,17-dione at least one mol reducing agent is used but not more than 5 mol reducing agent are used, and preferably 2 mol reducing agent.

**Patentansprüche**

1. Verfahren zur selektiven Reduktion eines 8,14-Seco-1,3,5(10),9(11)-oestratetraeen-14,17-dions zu dem entsprechenden 14,17-Ketol mittels eines chiralen Reduktionsmittel, dadurch gekennzeichnet, dass ein solches 14,17-Dion reduziert wird mit Hilfe eines Alkalimetallaluminiumhydridkomplex mit der Formel: $M\,Al(OR_1)_m(OR_2)_n\,H_{4-m-n}$, in der M ein Alkalimetall bedeutet; $OR_1$ abgeleitet ist von dem chiralen Alkohol (+)-(2S,3R)-4-Dimethylamino-3-methyl-1,2-diphenyl-2-butanol oder dessen Enantiomer mit der (−)-(2R,3S)-Konfiguration; $OR_2$ abgeleitet ist von einer achiralen organischen hydroxy-verbindung mit 1—10 C-atomen; M = 1—3; n = 0—2; und $m + n < 3$.

2. Verfahren nach Anspruch 1, dadurch gekennzeichnet dass M Lithium bedeutet.

3. Verfahren nach Anspruch 1 oder 2, dadurch gekennzeichnet dass ein 14,17-Dioxo-8,14-Secosteroid mit der Formel:

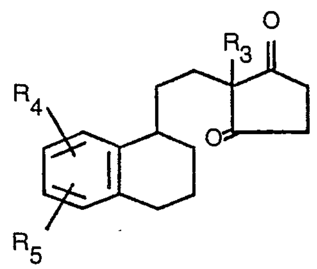

in der $R_3 = CH_3$ oder $C_2H_5$; $R_4$ = H, Alkyl, OH oder Alkoxy, und $R_5$ = H, Alkyl, OH oder Alkoxy, reduziert wird.

4. Verfahren nach Anspruch 3, dadurch gekennzeichnet, dass fuer $R_3 = CH_3$ das Diketon reduziert wird mit dem chiralen Reduktionsmittel, das beruht auf dem chiralen Alkohol mit der (+)-(2S,3R)-Konfiguration.

5. Verfahren nach Anspruch 3, dadurch gekennzeichnet, das fuer $R_3 = C_2H_5$ das Diketon reduziert wird mit dem chiralen Reduktionsmittel, das beruht auf dem chiralen Alkohol mit der (−)-(2R,3S)-Konfiguration.

6. Verfahren nach Ansprüchen 3—5, dadurch gekennzeichnet, dass $R_4$ = H und $R_3$ = 3-Methoxy.

7. Verfahren nach Ansprüchen 1—6, dadurch gekennzeichnet, dass n = 0.

8. Verfahren nach Ansprüchen 1—7, dadurch gekennzeichnet, dass m = 2—3, vorzugsweise 2—2.5 ist.

9. Verfahren nach Ansprüchen 1—8, dadurch gekennzeichnet, dass die Reduktion ausgefuhrt wird bei einer Temperatur zwischen −50°C und −100°C, vorzugsweise zwischen −60°C und −80°C.

10. Verfahren nach Ansprüchen 1—9, dadurch gekennzeichnet, dass per Mol 14,17-Dion wenigstens ein Mol Reduktionsmittel gebraucht wird, aber nicht mehr als 5 Mole Reduktionsmittel gebraucht werden, und vorzugsweise 2 Mole Reduktionsmittel.

**Revendications**

1. Procédé pour la réduction sélective d'une 8,14-seco-1,3,5(10),9(11)-oestratétraène-14,17-dione en le 14,17-cétol correspondant au moyen d'un agent réducteur chiral, charactérisé par le fait qu'on réduit une telle 14,17-dione à l'aide d'une complexe d'hydrure de métal alcalin et d'aluminium ayant pour formule: $M\ A1(OR_1)_m(OR_2)_n\ H_{4-m-n}$, dans laquelle M représente un métal alcalin; $OR_1$ dérive de l'alcool chiral (+)-(2S,3R)-4-diméthylamino-3-méthyl-1,2-diphényl-2-butanol ou de son énantiomère ayant la configuration (−)-(2R,3S); $OR_2$ dérive d'un composé hydroxylé organique non chiral ayant 1 à 10 atomes de carbone; m = 1—3; n = 0—2; et m + n≤3.

2. Procédé selon la revendication 1, caractérisé par le fait que M représente le lithium.

3. Procédé selon les revendications 1 ou 2, caractérisé par le fait que l'on réduit un 14,17-dioxo-8,14-seco-stéroïde ayant pour formule

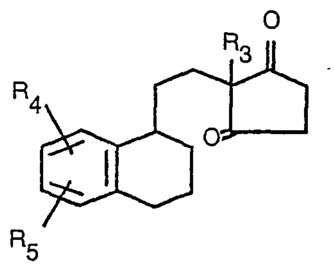

dans laquelle $R_3 = CH_3$ ou $C_2H_5$; $R_4$ = H, un alkyle, OH ou un alcoxy et $R_5$ = H, un alkyle, OH ou un alcoxy.

4. Procédé selon la revendication 3, caractérisé par le fait que pour $R_3 = CH_3$, on réduit la dicétone avec l'agent réducteur chiral à base de l'alcool chiral ayant la configuration (+)-(2S,3R).

5. Procédé selon la revendication 3, caractérisé par le fait que, pour $R_3 = C_2H_5$, on réduit la dicétone avec l'agent réducteur chiral à base de l'alcool chiral ayant la configuration (−)-(2R,3S).

6. Procédé selon les revendications 3 à 5, caractérisé par le fait que $R_4$ = H et $R_5$ = 3-méthoxy.

7. Procédé selon les revendications 1 à 6, caractérisé par le fait que n = o.

8. Procédé selon les revendications 1 à 7, caractérisé par le fait que m = 2—3, de préférence 2—2.5.

9. Procédé selon les revendication 1 à 8, caractérisé par le fait que la réduction est effectuée à une température entre −50°C et −100°C, de préférence entre −60°C et −80°C.

10. Procédé selon les revendications 1 à 9, caractérisé par le fait que, par mole de 14,17-dione, on utilise au moins une mole d'agent réducteur, mais on n'utilise pas plus de 5 moles d'agent réducteur et de préférence 2 moles d'agent réducteur.